# EUROPEAN PATENT APPLICATION

(11) **EP 3 583 936 A1**
(43) Date of publication of application: **25.12.2019**
(21) Application number: 19180471.5
(22) Date of filing: 17.06.2019
(51) Int. Cl.: A61K 9/16, A61K 9/20, A61K 9/50, A61K 31/366, A61K 31/397

(54) **COMBINED PHARMACEUTICAL COMPOSITION FOR THE TREATMENT OF DYSLIPIDEMIA AND METHOD OF MANUFACTURE THEREOF**

(30) Priority: 19.06.2018 PL 42597518
(71) Applicant: Invest Bielany Spolka z ograniczona odpowiedzialnoscia, 04-028 Warszawa (PL)
(72) Inventor: RAPACZ, Joanna, 05-400 Otwock (PL); NAGAJ, Justyna, 53-030 Wroclaw (PL); URBANSKA, Agnieszka, 05-110 Jablonna (PL); IGNACIUK, Aneta, 05-119 Lajski (PL); WISNIEWSKA, Anna, 01-873 Warszawa (PL); NAWROCKI, Ryszard, 03-131 Warszawa (PL)
(74) Representative: BRANDPAT Patent and Trademark Attorneys Chlebicka Czyz Galazkiewicz Ziolkowski Professional Partnership

(57) **Abstract**

The present invention relates to a combined pharmaceutical composition for the treatment of dyslipidemia containing ezetimibe and simvastatin in pharmaceutically effective amounts, in the form of a tablet containing ezetimibe with particle size d(0.9) below 10 µm, simvastatin with particle size d(0.9) below 15 µm, lactose, microcrystalline cellulose, croscarmellose sodium, hypromellose, citric acid, butylated hydroxyanisole, propyl gallate and magnesium stearate as a glidant, wherein lactose is present in the composition in the amount of above 67% by weight and the ratio of microcrystalline cellulose to lactose is from 1:4 to 1:5.

## Description

### FIELD OF THE INVENTION

The present invention relates to a combined pharmaceutical composition for the treatment of dyslipidemia containing ezetimibe and simvastatin, and method of manufacture of such composition.

### BACKGROUND OF THE INVENTION

Ezetimibe is an active ingredient that lowers cholesterol blood level. It acts by lowering cholesterol absorption in the small intestine. It may be used separately or in combination with HMG-CoA reductase inhibitors (statins), when statins alone are ineffective in controlling cholesterol levels. Ezetimibe makes up a new class of lipid-lowering compounds, which inhibit absorption of cholesterol and related plant sterols in the gastrointestinal tract. Ezetimibe is active following oral administration and has a mechanism of action different than other classes of cholesterol level-lowering compounds (e.g. statins, bile acids binding agents (resins), fibric acid derivatives, plant stanols). The molecular target for ezetimibe is a sterol transporter, Niemann-Pick C1-like 1 (NPC1L1) protein, which is responsible for the uptake of cholesterol and phytosterols in the gastrointestinal tract.

Ezetimibe binds with the brush border of the small intestine and inhibits cholesterol absorption, thus leading to the reduction in the amount of cholesterol transported to the liver; statins reduce cholesterol synthesis in the liver and the combination of these two mechanisms reduces cholesterol level in a complementary manner. In a 2-week clinical trial involving 18 subjects with hypercholesterolemia ezetimibe inhibited cholesterol absorption from the gastrointestinal tract by 54% compared with placebo.

A series of non-clinical trials has been conducted to determine whether the effect of ezetimibe inhibiting cholesterol absorption is selective. Ezetimibe inhibited the absorption of cholesterol labelled with ¹⁴C carbon isotope, without any effects on absorption of triglycerides, fatty acids, bile acids, progesterone, ethinyl estradiol or fat-soluble vitamins A and D. In epidemiological studies it has been determined that cardiovascular morbidity and mortality is proportional to the levels of total cholesterol (total-C) and LDL-C, and inversely proportional to HDL-C level. Ezetimibe administration with statin is effective in the reduction of the risk of cardiovascular events in patients with coronary arterial disease and acute coronary syndrome episode.

In controlled clinical trials, ezetimibe both as monotherapy, and co-administered with statin significantly reduced total cholesterol (total-C), low-density cholesterol (LDL-C), apolipoprotein B (Apo B) and triglyceride (TG) level and increased the level of high-density cholesterol (HDL-C) in patients with hypercholesterolemia.

Simvastatin is an HMG-CoA reductase inhibitor. Following oral administration, simvastatin, which is an inactive lactone, is hydrolyzed in the liver to a form of a corresponding, active beta-hydroxyacid, which is a strong HMG-CoA reductase inhibitor (3-hydroxy-3-methylglutaryl-coenzym A reductase). This enzyme catalyzes HMG-CoA conversion into mevalonate, which is an early and transition rate-limiting stage in cholesterol biosynthesis.

It was demonstrated that simvastatin causes reduction of both normal and increased LDL-C cholesterol level. LDL consists of very low density proteins (VLDL) and is catabolised mostly through an LDL receptor of very high affinity. The mechanism of reduction of LDL cholesterol level by simvastatin may result both from reducing VLDL cholesterol concentration (VLDL-C), and induction of LDL receptor that leads to the reduced production and increased catabolism of LDL-C cholesterol. During simvastatin treatment the apolipoprotein B concentration becomes significantly reduced. Moreover, simvastatin causes slight increase in HDL cholesterol concentration and reduction of TG concentration in plasma. Resulting from these effects, the ratios of total cholesterol to HDL cholesterol, and LDL cholesterol to HDL cholesterol are reduced.

The formulation containing ezetimibe and simvastatin is a lipid level-lowering product that selectively inhibits absorption of cholesterol and related plant sterols from the gastrointestinal tract, as well as inhibits endogenous cholesterol synthesis.

### BRIEF DESCRIPTION OF DRAWINGS

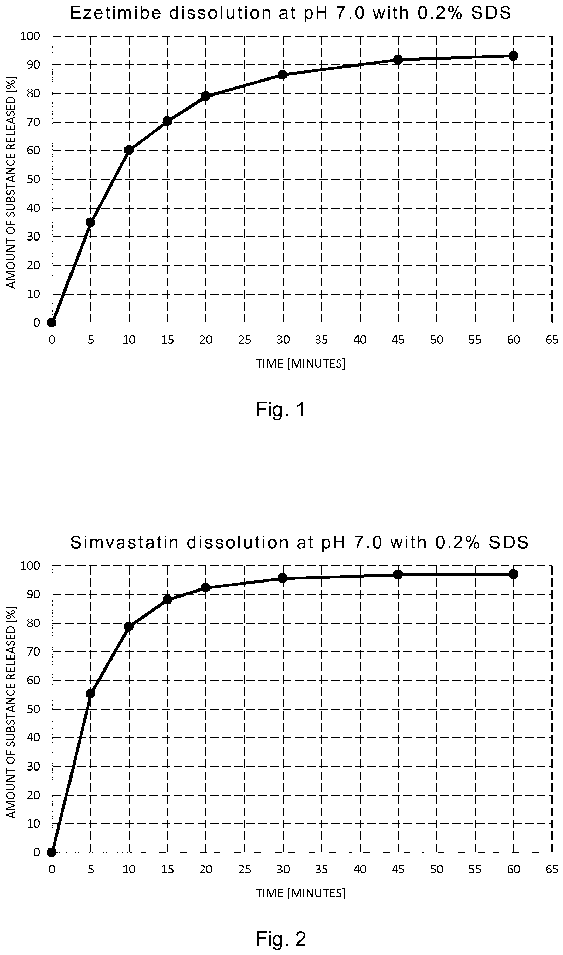

Fig. 1. shows ezetimibe dissolution profile in tablets of Example 1 at pH 7.0 with 0.2% SDS.
Fig. 2. shows simvastatin dissolution profile in tablets of Example 1 at pH 7.0 with 0.2% SDS.

### SUMMARY OF THE INVENTION

Both ezetimibe and simvastatin are substances practically insoluble within the physiological pH range. The object of the present invention was to obtain a combination formulation of ezetimibe and simvastatin with high bioavailability of both active ingredients. The object was achieved by choosing the appropriate particle size of active ingredients and combination of excipients including: lactose, microcrystalline cellulose, croscarmellose sodium, hypromellose, citric acid, butylated hydroxyanisole, propyl gallate and magnesium stearate. It has been found that reduced proportion of microcrystalline cellulose in the composition, at the expense of increased lactose proportion, accelerates the initial (for the first 15 minutes) dissolution rate of ezetimibe and simvastatin from a tablet, due to immediate tablet disintegration. On the other hand, increased proportion of microcrystalline cellulose at the expense of the reduced lactose proportion, causes that dissolution profile at 20 minutes is lowered and total ezetimibe dissolution level at 60 minutes is low (approximately 80% ezetimibe dissolved). This phenomenon may be explained by the fact that very fine ezetimibe particles d(0.9) <10 µm adhere to hydrophobic microcrystalline cellulose particles, causing a reduction in dissolution profile. Unexpectedly, it appeared that rapid and complete ezetimibe and simvastatin dissolution was achieved for a composition with a high lactose content at a proper ratio of microcrystalline cellulose to lactose.

Simvastatin decomposes easily as a result of oxidation. Therefore, three antioxidants were used to obtain a stable composition. In the tablets of the invention citric acid, butylated hydroxyanisole and propyl gallate in effective amounts were used as antioxidants. Without the above-mentioned antioxidants simvastatin degradation is observed. Preferably, stabilized simvastatin mixed with butylated hydroxyanisole is used as a raw material for the preparation of the composition of the present invention.

### DETAILED DESCRIPTION OF THE INVENTION

In the first aspect, the present invention provides immediate release, stable, combined pharmaceutical formulation in a form of a tablet containing ezetimibe and simvastatin in pharmaceutically effective amounts. Preferably, the invention provides tablets containing ezetimibe with particle size d(0.9) below 10 µm, simvastatin with particle size d(0.9) below 15 µm, lactose, microcrystalline cellulose, croscarmellose sodium, hypromellose, citric acid, butylated hydroxyanisole, propyl gallate and magnesium stearate as a glidant, wherein lactose is present in the composition in the amount of more than 67% and the ratio of microcrystalline cellulose to lactose is from 1:4 to 1:5.

In the first embodiment, the ratio of microcrystalline cellulose to lactose in the tablet of the invention is 1:4.

In another embodiment, the ratio of microcrystalline cellulose to lactose in the tablet of the invention is 1:5.

In a preferred embodiment, the ratio of microcrystalline cellulose to lactose in the tablet of the invention is 1:4.5.

In the present description the ratio of microcrystalline cellulose to lactose is specified as a weight ratio.

In the most preferred embodiment lactose is present in the composition in the amount of above 67% and below 72%.

In a preferred embodiment the composition of the invention is in the form of a tablet manufactured by wet granulation and compression using a tablet press. The tablet of the present invention releases at least 85% ezetimibe and at least 85% simvastatin within 45 minutes in the medium of pH 7.0 with 0.2% SDS (sodium lauryl sulphate) in a paddle apparatus at 50 rpm at 37.0 ± 0.5°C.

Dissolution studies presented herein have been carried out using buffer solutions as specified in the European Pharmacopoeia 9.0, i.e. using methodology as per the European Pharmacopoeia 9.0, chapter 2.9.3 "Dissolution test for solid dosage forms" in a Type 2 apparatus (paddle apparatus). 900 ml of medium was used in each vessel. Content of the active substances ezetimibe and simvastatin was determined by HPLC using UV/VIS detector. Percent dissolution of the active substance was calculated as a mean of 6 consecutive dissolution experiments. Ezetimibe and simvastatin are practically insoluble in buffers: pH 1.2, pH 4.5 and pH 7.0, therefore to achieve sink conditions as required in dissolution tests, it was necessary to add a surfactant to the dissolution medium.

In a preferred embodiment ezetimibe particle size distribution is represented by d(0.9) below 10 µm, meaning that 90% ezetimibe particles, by volume, have a diameter below 10 µm. In a preferred embodiment simvastatin particle size distribution is represented by d(0.9) below 15 µm, meaning that 90% simvastatin particles, by volume, have a diameter below 15 µm. The particle size distribution is typically reported as a cumulative undersize distribution. The symbol d is used to denote particle size, which in turn is defined as a diameter of a volume-equivalent sphere. d(0.9) denotes a volume fraction undersize at the particle size d. Particle sizes at the undersize values of 10%, 50% and 90% volume fraction are denoted as d(0.1), d(0.5) and d(0.9), respectively.

Ezetimibe and simvastatin particle size was measured by laser diffraction method described in the European Pharmacopoeia 9.0, chapter 2.9.31 "Particle size analysis by laser light diffraction" using Mastersizer 2000 apparatus with Hydro 2000 S adapter.

The tablet of the present invention contains ezetimibe and simvastatin in a pharmaceutically effective amounts. The content of each of the active substances refers to an anhydrous substance, unless otherwise indicated.

The term "lactose" as used herein refers to any type of lactose, suitable for pharmaceutical purposes, such as anhydrous lactose, lactose monohydrate, screened lactose, ground lactose, spray-dried lactose, granulated lactose. Preferably, lactose monohydrate is used to manufacture the tablets of the present invention.

The term "citric acid" as used herein refers to any type of citric acid, suitable for pharmaceutical purposes, such as anhydrous citric acid and citric acid monohydrate. Preferably, citric acid monohydrate is used to manufacture the tablets of the present invention.

In one embodiment, the tablet of the present invention contains 10 mg ezetimibe and 10 mg simvastatin as active ingredients.

In another embodiment, the tablet of the present invention contains 10 mg ezetimibe and 20 mg simvastatin as active ingredients.

In another embodiment, the tablet of the present invention contains 10 mg ezetimibe and 40 mg simvastatin as active ingredients.

Preferably, the tablet of the present invention contains, in weight per cent:
- from 1.2% to 1.3% ezetimibe,
- from 1.2% to 5.0% simvastatin,
- above 67% and below 72% lactose,
- 15% microcrystalline cellulose,
- 8% croscarmellose sodium,
- 2% hypromellose,
- from 0.1% to 0.2% citric acid,
- 0.01% butylated hydroxyanisole,
- 0.0025% propyl gallate,
- from 1.0 to 1.5% magnesium stearate.

Preferably, the weight of an individual tablet of the invention is from 608 mg to 1013 mg, especially preferably the mean tablet weight is 810 mg.

In the second aspect, the present invention provides a method of manufacture of a tablet containing ezetimibe, simvastatin, lactose, microcrystalline cellulose, croscarmellose sodium, hypromellose, citric acid, butylated hydroxyanisole, propyl gallate and magnesium stearate as a glidant, wherein the lactose is present in the composition in an amount above 67% and the ratio of microcrystalline cellulose to lactose is from 1:4 to 1:5, wherein the method comprises the following steps:
a) mixing ezetimibe with a particle size distribution defined as d(0.9) below 10 µm and simvastatin with a particle size distribution defined as d(0.9) below 15 µm, as well as butylated hydroxyanisole with lactose, croscarmellose sodium and hypromellose;
b) adding microcrystalline cellulose to the blend obtained in step a) and mixing;
c) wet granulation of the blend obtained in step b), after which calibration and drying of the obtained granulate is carried out;
d) adding magnesium stearate to the granulate obtained in step c) and mixing;
e) compressing the final blend obtained in step d) to form a tablet.

The invention has been illustrated by the following examples, which are not limiting the scope of protection of the present invention.

### Examples

### Example 1

Method of manufacture: All raw materials were weighed out in accordance with the composition specified in the table below.

| **No.** | **Raw material** | **Quantity [kg]** |
|---|---|---|
| 1. | Ezetimibe | 0.60 |
| 2. | Simvastatin | 0.60 |
| 3. | Lactose monohydrate | 34.48 |
| 4. | Microcrystalline cellulose | 7.28 |
| 5. | Croscarmellose sodium | 3.89 |
| 6. | Hypromellose | 0.96 |
| 7. | Citric acid monohydrate | 0.06 |
| 8. | Butylated hydroxyanisole | 0.0048 |
| 9. | Propyl gallate | 0.0012 |
| 10. | Magnesium stearate | 0.72 |
| | Total batch size | 48.60 |

Ezetimibe with a particle size distribution defined as d(0.9) below 10 µm and simvastatin with a particle size distribution defined as d(0.9) below 15 µm were mixed in a high-shear granulator with lactose monohydrate, croscarmellose sodium and hypromellose to give blend I. Simvastatin was used as a raw material, which was stabilized by adding an antioxidant - butylated hydroxyanisole. In the next step the microcrystalline cellulose was added to the above-mentioned granulator and mixed, to give blend II. Granulation of blend II was performed in a high-shear granulator. Granulating solution was supplied for 60 - 90 seconds. After the granulating solution was supplied, the granulation process was continued up to 180 seconds. Rotational speed of the bottom stirrer was the same throughout the whole granulation process. The wet granulate was calibrated through a 1.5 mm sieve. Following calibration, the wet granulate was dried. The drying process was continued until the moisture content in the granulate was below 4.0%. After the drying on a fluidized bed was finished, the granulate was calibrated.

Magnesium stearate was added to the tank with the granulate and mixed.

The tablet compression process was carried out using a rotary tablet press.

One tablet contained 10 mg ezetimibe and 10 mg simvastatin and its total weight was 810 mg.

70.4% ezetimibe and 88.2% simvastatin was released from the obtained tablets within 15 min. in the medium of pH 7.0 with 0.2% SDS using a paddle apparatus at 50 rpm and at 37.0 ± 0.5°C. Moreover, 91.8% ezetimibe and 97.0% simvastatin was released within 45 minutes under the same dissolution conditions.

### Example 2

The same method of manufacture as in Example 1 was used, but one tablet contained 10 mg of ezetimibe and 20 mg of simvastatin and its weight was 810 mg. The content of the combination used to manufacture the tablets is presented in the table below:

| **No.** | **Raw material** | **Quantity [kg]** |
|---|---|---|
| 1. | Ezetimibe | 0.60 |
| 2. | Simvastatin | 1.20 |
| 3. | Lactose monohydrate | 33.88 |
| 4. | Microcrystalline cellulose | 7.28 |
| 5. | Croscarmellose sodium | 3.89 |
| 6. | Hypromellose | 0.96 |
| 7. | Citric acid monohydrate | 0.06 |
| 8. | Butylated hydroxyanisole | 0.0048 |
| 9. | Propyl gallate | 0.0012 |
| 10. | Magnesium stearate | 0.72 |
| | Total batch size | 48.60 |

71.5% ezetimibe and 90.3% simvastatin was released from the obtained tablets within 15 min. in the medium of pH 7.0 with 0.2% SDS using a paddle apparatus at 50 rpm and at 37.0 ± 0.5°C. Moreover, 91.1% ezetimibe and 102.4% simvastatin was released within 45 minutes under the same dissolution conditions.

### Example 3

The same method of manufacture as in Example 1 was used, but one tablet contained 10 mg of ezetimibe and 40 mg of simvastatin and its weight was 810 mg. The content of the composition used to manufacture the tablets is presented in the table below:

| **No.** | **Raw material** | **Quantity [kg]** |
|---|---|---|
| 1. | Ezetimibe | 0.60 |
| 2. | Simvastatin | 2.40 |
| 3. | Lactose monohydrate | 32.68 |
| 4. | Microcrystalline cellulose | 7.28 |
| 5. | Croscarmellose sodium | 3.89 |
| 6. | Hypromellose | 0.96 |
| 7. | Citric acid monohydrate | 0.06 |
| 8. | Butylated hydroxyanisole | 0.0048 |
| 9. | Propyl gallate | 0.0012 |
| 10. | Magnesium stearate | 0.72 |
| | Total batch size | 48.60 |

71.7% ezetimibe and 89.5% simvastatin was released from the obtained tablets within 15 min. in the medium of pH 7.0 with 0.2% SDS using a paddle apparatus at 50 rpm and at 37.0 ± 0.5°C. Moreover, 91.3% ezetimibe and 100.3% simvastatin was released within 45 minutes under the same dissolution conditions.

## Claims

1. Combined pharmaceutical composition containing ezetimibe and simvastatin in pharmaceutically effective amounts, **characterized in that** it is in the form of a tablet containing ezetimibe with particle size d(0.9) below 10 µm, simvastatin with particle size d(0.9) below 15 µm, lactose, microcrystalline cellulose, croscarmellose sodium, hypromellose, citric acid, butylated hydroxyanisole, propyl gallate and magnesium stearate as a glidant, wherein lactose is present in the composition in the amount of above 67% by weight and the ratio of microcrystalline cellulose to lactose is from 1:4 to 1:5.

2. Combined pharmaceutical composition according to claim 1, **characterized in that** it releases at least 85% ezetimibe and at least 85% simvastatin within 45 minutes in the medium of pH 7.0 with 0.2% SDS in a paddle apparatus at 50 rpm at 37.0 ± 0.5°C.

3. Combined pharmaceutical composition according to claim 1 or 2, **characterized in that** the ratio of microcrystalline cellulose to lactose is 1:4.

4. Combined pharmaceutical composition according to claim 1 or 2, **characterized in that** the ratio of microcrystalline cellulose to lactose is 1:5.

5. Combined pharmaceutical composition according to claim 1 or 2, **characterized in that** the ratio of microcrystalline cellulose to lactose is 1:4.5.

6. Combined pharmaceutical composition according to any of claims 1 - 5, **characterized in that** lactose is present in the composition in the amount of above 67% and below 72% by weight.

7. Combined pharmaceutical composition according to any of claims 1 - 6, **characterized in that** lactose has a form of lactose monohydrate.

8. Combined pharmaceutical composition according to any of claims 1 - 7, **characterized in that** citric acid has a form of citric acid monohydrate.

9. Combined pharmaceutical composition according to claim 1, containing from 1.2% to 1.3% ezetimibe, from 1.2% to 5.0% simvastatin, above 67% and below 72% lactose monohydrate, 15% microcrystalline cellulose, 8% croscarmellose sodium, 2% hypromellose, from 0.1% to 0.2% citric acid monohydrate, 0.01% butylated hydroxyanisole, 0.0025% propyl gallate, and from 1.0 to 1.5% magnesium stearate.

10. Combined pharmaceutical composition according to any of claims 1 - 9, **characterised in that** it is in the form of a tablet manufactured by wet granulation and compression using a tablet press.

11. Combined pharmaceutical composition according to any of claims 1 - 10, **characterised in that** it contains 10 mg of ezetimibe and 10 mg of simvastatin as active substances.

12. Combined pharmaceutical composition according to any of claims 1 - 10, **characterised in that** it contains 10 mg of ezetimibe and 20 mg of simvastatin as active substances.

13. Combined pharmaceutical composition according to any of claims 1 - 10, **characterised in that** it contains 10 mg of ezetimibe and 40 mg of simvastatin as active substances.

14. Combined pharmaceutical composition according to any of claims 1 - 10, **characterised in that** the weight of an individual tablet is from 608 mg to 1013 mg, especially preferably the mean tablet weight is 810 mg.

15. The method of manufacture of a tablet containing ezetimibe, simvastatin, lactose, microcrystalline cellulose, croscarmellose sodium, hypromellose, citric acid, butylated hydroxyanisole, propyl gallate and magnesium stearate as a glidant, wherein lactose is present in the composition in the amount of above 67% and the ratio of microcrystalline cellulose to lactose is from 1:4 to 1:5, wherein the method includes the following steps:
a) mixing ezetimibe with particle size distribution defined as d(0.9) below 10 µm and simvastatin with particle size distribution defined as d(0.9) below 15 µm, as well as butylated hydroxyanisole with lactose, croscarmellose sodium and hypromellose;
b) adding microcrystalline cellulose to the blend obtained in step a) and mixing;
c) wet granulation of the blend obtained in step b), followed by calibration and drying of the obtained granulate;
d) adding magnesium stearate to the granulate obtained in step c) and mixing;
e) compressing the final blend obtained in step d) to form a tablet.
